# EUROPEAN PATENT APPLICATION

(11) **EP 3 593 650 A2**
(43) Date of publication of application: **15.01.2020**
(21) Application number: 19179829.7
(22) Date of filing: 12.06.2019
(51) Int. Cl.: A23L 29/00, A23L 2/52, A23L 2/68, A61K 33/00, A61K 9/00, C01B 3/00, A23L 2/39, A61P 39/06

(54) **PREPARATIONS CAPABLE OF INCREASING THE PH OF FOOD BY RELEASING MOLECULAR HYDROGEN**

(30) Priority: 19.06.2018 IT 201800006414
(71) Applicant: Vivere Alcalino S.r.l., 72018 San Michele Salentino (BR) (IT)
(72) Inventor: PALMISANO, Rocco, 72018 San Michele Salentino (BR) (IT)
(74) Representative: Bruni, Giovanni

(57) **Abstract**

Preparation according to claim 1, for liquid or powdered food use, capable of increasing the pH of food in which it is added, by releasing molecular hydrogen in the presence of water, comprising a mixture of the following compounds: potassium carbonate, potassium sulfate, dibasic potassium phosphate, sodium carbonate, calcium chloride, magnesium oxide.

## Description

### Technical field of the invention

The present invention relates to new preparation for acidity regulation, i.e. to mixtures of substances with the aim of maintaining their pH within certain intervals and their use in food and beverages. In particular such new preparation are capable of releasing molecular hydrogen (H₂) when some components of the mixture come in contact with water. Advantageously, the preparations object of the present invention can conveniently be added to beverages and/or food in order to correct their pH and immediately obtain the benefits produced by the release of H₂ in the human body in addition to the anti-acid buffer effect.

### Known art

According to the state of the art many publications and scientific studies are known regarding molecular hydrogen, which has been shown to have excellent antioxidant and anti-inflammatory properties. Numerous publications are available in the literature that describe its biological and medical benefits; for example, it was discovered that H₂ reduces the oxidative stress not only through direct reactions with strong oxidants, but also indirectly by adjusting various gene expressions. In addition, H₂ works as an anti-inflammatory and anti-apoptotic and stimulates metabolic energy.

For example, the publication of Ohta S., "Molecular hydrogen as a preventive and therapeutic medical gas: initiation, development and potential of hydrogen medicine" PharmacolTher, 2014, shows that H₂ reacts with strong oxidants such as hydroxyl radical in cells and has proposed its potential for preventive and therapeutic applications. H₂ also has a series of advantages: it spreads rapidly in tissues and cells, and is gentle enough in order neither to disturb the metabolic redox reactions nor to influence the signaling of the reactive species in oxygen.

The publication of Ignacio, RM, et al., "The Drinking Effect of Hydrogen Water on Atopic Dermatitis Induced by Dermatophagoidesfarinae Allergen in NC/Nga Mice" EvidBasedComplementAlternatMed, 2013, demonstrates that active hydrogen acts as an antioxidant and shows the protection of the oxidative-induced damage in vitro. It has also been reported that it can selectively select reactive oxygen species (ROS) and has shown a positive influence on the imbalance of cytokines. It is also known that hydrogen easily penetrates the skin, spreads rapidly in tissues and cells and is distributed in the body through the bloodstream. H₂ can be incorporated into the body through inhalation of H₂ gas, by drinking water with dissolved H₂ and by injection of saline solution with dissolved H₂. The water produced by the electrolysis of water is included in drinking water containing a high concentration of hydrogen gas and has a property of a high level of dissolved hydrogen (DH), a negative oxidation reduction potential (ORP) and an alkaline pH. Previous studies on electrolytic reduced water (ERW) and reduced alkaline water, which are also hydrogen-rich waters produced by the same reaction with HW, have shown various beneficial effects such as the reduction of triglyceride and blood sugar levels in the OLEFT diabetic rat model, the prevention of insulin resistance and inflammation of the liver. Furthermore, oral intake of hydrogen-rich water has shown an anti-allergic effect in vivo.

According to the state of the art however are not known preparations capable to correct and/or to increase the pH of food by means of simply adding them in aqueous solutions/beverages (ie water, coffee, milk, infusions, tea, herbal teas, juices of various kinds and wine) as well as in food having a liquid and/or powder shape/state (ie chocolate, candy, condiments, ice cream and jellies, integrators, honey, soups, sauces and flour for bakery and pastry products) and at same time they release H₂ when the mixture comes into contact with water.

### Summary of the invention

Purpose of the present invention is to provide new preparations for the correction of pH, which can be easily added together with food and beverages of normal consumption, which in addition release H₂ in presence of water. In particular, the preparations object of the present invention comprise mixtures of chemicals which amplify the possible antioxidant effect still present in the products to which the formulation is added.

Molecular hydrogen, a notoriously unstable gas, with the present formulation can remain available for days and, in many cases, for weeks if the treated product is stored indoors or under vacuum.

According to an aspect of the present invention, some preparations are described having the characteristics set forth in the annexed independent product claim.

Further preferred and/or particularly advantageous embodiments of the invention are described according to the characteristics set forth in the annexed dependent claims.

### Detailed description

According to the invention, the new preparations comprise a mixture of acidity regulators, ie capable of increasing the pH of food to which they are added, in powder or liquid form, to be diluted or added to food and/or beverages in variable concentrations depending on the solutions and food to be treated.

A first preparation for food use in liquid or powder state, capable of increasing the pH of food to which it is added by releasing molecular hydrogen in the presence of water, comprises a mixture of the following compounds:
- potassium carbonate
- potassium sulfate
- dibasic potassium phosphate
- sodium carbonate
- calcium chloride
- magnesium oxide.

Advantageously, to ensure greater stability, said compounds are used in the following weight percentages:
- 35-40% potassium carbonate
- 20-25% potassium sulphate
- 3-5% dibasic potassium phosphate
- 15-20% sodium carbonate
- 3-7% calcium chloride
- 1-3% magnesium oxide.

Advantageously, a variable quantity according to the type of food/beverage of Vitamin C and of other acids (fumaric acid, tartaric acid, etc.) for the preparation of food integrators can be added to the preparation as described . In addition, such preparation can be used both in powder form, in capsules, in tablets and in liquid form (drops) to be added to beverages under treatment.

A second preparation for food use in liquid or powder form, capable of increasing the pH of food to which it is added by releasing molecular hydrogen in the presence of water, comprises a mixture of the following main compounds:
- potassium hydroxide
- ascorbic acid
- magnesium gluconate and
- sodium chloride

Advantageously, in order to ensure greater stability, said compounds are used in the following weight percentages, considering a powder formulation on 100 g, as follows:
- 25-35% potassium hydroxide
- 20-30% ascorbic acid
- 5-15% magnesium gluconate
- 5-10% sodium chloride.

Advantageously, the powder formulation further comprises, considering a powder formulation on 100 g, further compounds with the following weight percentages:
- 5-15% calcium ascorbate
- 5-15% potassium sulphate
- 10% sodium hydroxide
- 3-10% tartaric acid
- 1-5% magnesium hydroxide.

The above-mentioned compounds can be used in any combination with each other but are mixed according to any percentage between those indicated, obviously being their sum equal to 100%.

The preparation is obtained by simple and gentle mixing by stirring the compounds. In the preparation of the powder first magnesium hydroxide is introduced and then by means of mixing and slow stirring with plastic blades made in plastic or glass containers, in the following order: tartaric acid, sodium chloride, magnesium gluconate, potassium sulfate, calcium ascorbate, sodium hydroxide, potassium hydroxide, ascorbic acid.

Such preparation must not be used as it is, but it must be used in solutions or in liquefied food. The present formulation can also be used by suitably dosing it through measuring means or pre-dosed capsules, which are not suitable for being directly ingested, but only for the purpose of being comfortably used according to the manufacturer's indications on the basis of the product to be treated: water, infusions, herbal teas, coffee, chocolate, etc.

As the dosing varies according to the substance under treatment, the user can choose the desired effect within the legal security parameters by measuring the pH and/or H₂ which are evident in the preparation to be taken.

In experimental tests with water 0,5 g of the preparation object of the present invention were obtained, 0,5-0,6 ppm of molecular hydrogen and a pH around 10. By using instead dark chocolate with 1-2 g of the preparation of the present invention in 100 g of dark chocolate, an increase in pH of 1,5 units was obtained, that is from a value of pH 5,5-6 to a value of pH 7-7,5. By using honey, with 2 g of preparation 100 g of honey, an increase in the pH from 4,5-5 to 7,5-8 is obtained. By using coffee powder with 1-2 g of preparation on 100 g of coffee, a final pH from 7,5 to 7,8 was obtained. Obviously the doses change according to the basic acidity of the product under treatment.

In an alternative embodiment, the second preparation can also occur in the liquid state. In this case, the volumetric percentages on 100 ml of said compounds are:
- 18-28% potassium hydroxide
- 5-10% sodium chloride
- 1-2% ascorbic acid
- 0,5-2% magnesium gluconate.

Preferably, the liquid formulation also comprises a further compound with following volume percentages:
- 0,25-0,50% citric acid,
- water enough for 100 ml.

Also in this case, the use of the preparation in the liquid state must always be diluted and never used as such. The dosage of the present preparation depends on the liquid under treatment, and therefore the user will have to comply with the directions of the supplier/manufacturer who will suggest the optimum amount to be used for each substance with maximum health safety and maximum benefit.

Advantageously, use of the liquid preparation may be administered in drops depending on the type of beverage under treatment.

The preparation in liquid form is obtained by simple mixing and stirring the compounds in plastic, glass or steel containers starting with the volume of water and, following with the present product order: sodium chloride, potassium hydroxide, ascorbic acid, magnesium gluconate, citric acid. The proportions should be stored under penalty of ineffectiveness of the release of H₂, whereas the greater critical point is the ratio of potassium component and ascorbic acid, citric acid and tartaric acid.

The production of hydrogen is mainly dependent on the chemical reaction between alkaline buffers (K, Na, Ca, Mg) present in the mixture and ascorbic acid. As can be seen from some of the formulas shown below, the aforementioned alkaline buffers react with hydrogen (which is mainly present in ascorbic acid) by substituting themselves to the latter, and thus by freeing molecular hydrogen which remains in water for days and is stored in tightly closed containers.

| **ASCORBIC ACID** | **POTASSIUM HYDROXIDE** |
|---|---|
| 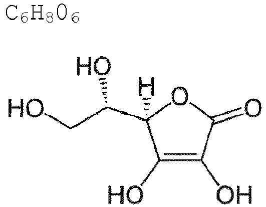 | KOH |
| | K - O - H |

| **SODIUM HYDROXIDE** | **TARTARIC ACID** |
|---|---|
| NaOH | 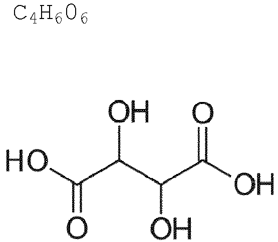 |
| Na-O-H | |

| **CALCIUM ASCORBATE** | **MAGNESIUM GLUCONATE** |
|---|---|
| 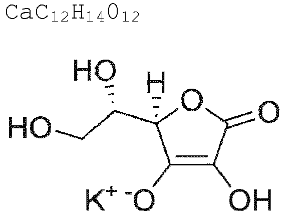 | 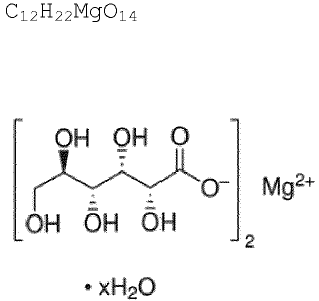 |

The above formulae are to be classified as acidity correctors, and the same can be classified as integrators by adding the due quantities, for example, of selenium, chrome, boron, zinc, etc.

In addition to the embodiments of the invention, as described above, it is to be understood that there are numerous further variants. It must also be understood that said embodiments are only exemplary and do not limit either the object of the invention, or its applications, or its possible configurations. On the contrary, although the above description makes it possible for the man of the trade to carry out the present invention at least according to its exemplary configuration, it is to be understood that many variations of the described components are conceivable, without thereby escaping from the object of the invention, as defined in the attached claims, literally interpreted and/or according to their legal equivalents.

## Claims

1. Preparation according to claim 1, for liquid or powdered food use, capable of increasing the pH of food in which it is added, by releasing molecular hydrogen in the presence of water, comprising a mixture of the following compounds:
- potassium carbonate
- potassium sulfate
- dibasic potassium phosphate
- sodium carbonate
- calcium chloride
- magnesium oxide.

2. Preparation according to claim 1, wherein the weight percentages of said compounds are:
- 35-40% potassium carbonate
- 20-25% potassium sulphate
- 3-5% dibasic potassium phosphate
- 15-20% sodium carbonate
- 3-7% calcium chloride
- 1-3% of magnesium oxide.

3. Use of the preparation according to claim 1 or 2, **characterized by** the addition of Vitamin C for the preparation of food integrators.

4. Use of the preparation according to claims 1 or 2, for the production of capsules of specific dosage or in powder form for industrial use.

5. Use of the preparation according to claims 1 or 2 for using the liquid preparation in drops according to the type of beverage under treatment.

6. Preparation for food use in liquid or powder state, capable of increasing the pH of food in which it is added, comprising a mixture of the following compounds:
- potassium hydroxide
- ascorbic acid
- magnesium gluconate and
- sodium chloride
**characterized in that** said preparation releases molecular hydrogen in the presence of water.

7. Preparation according to claim 6, wherein in the powder formulation the weight percentages of said compounds are:
- 25-35% potassium hydroxide
- 20-30% ascorbic acid
- 5 -15% magnesium gluconate
- 5-10% sodium chloride.

8. Preparation according to claim 7, wherein the powder formulation further comprises additional compounds with the following weight percentages:
- 5-15% calcium ascorbate
- 5-15% potassium sulphate
- 3-10% sodium hydroxide
- 3-10% tartaric acid
- 1 -5% magnesium hydroxide .

9. Preparation according to claim 6, where in the formulation in the liquid state the volume percentages of said compounds are:
- 18-28% potassium hydroxide
- 1-2% ascorbic acid
- 0,5-2% magnesium gluconate
- 5-10% sodium chloride
- water enough for 100 ml.

10. Preparation according to claim 9, wherein the formulation in the liquid state also comprises an additional compound with volume percentages:
- 0,25-0,50% citric acid,
- water enough for 100 ml.

11. Use of the preparation according to claims 7 and 8, for the production of capsules and of specific dosage or in powder form for industrial use.

12. Use of the preparation according to claims 9 and 10 for the use of the liquid preparation in drops according to the type of beverage under treatment.
